# EUROPÄISCHE PATENTSCHRIFT

(11) **EP 3 782 664 B1**
(45) Veröffentlichungstag und Bekanntmachung des Hinweises auf die Patenterteilung: **03.12.2025**
(21) Anmeldenummer: 20190367.1
(22) Anmeldetag: 10.08.2020
(51) Int. Cl.: A61M 1/02, A61M 1/36, B01L 3/00

(54) **VORRICHTUNG UND VERFAHREN ZUM SEPARIEREN VON BLUT**
DEVICE AND METHOD FOR SEPARATING BLOOD
DISPOSITIF ET PROCÉDÉ DE SÉPARATION DU SANG

(30) Priorität: 11.08.2019 DE 102019121614
(43) Veröffentlichungstag der Anmeldung: 24.02.2021
(73) Patentinhaber: Yapici, Ahmet, 07070 Konyaaltý/Antalya (TR)
(72) Erfinder: Yapici, Ahmet, 07070 Konyaaltý/Antalya (TR)
(74) Vertreter: Patentanwaltzkanzlei Hutzelmann

(56) Entgegenhaltungen:
- EP-A2- 2 495 302
- AU-A1- 2013 312 546
- GB-A- 1 385 576
- US-A1- 2014 371 048
- US-A1- 2015 104 824

## Beschreibung

Die Erfindung bezieht sich auf eine Vorrichtung und ein Verfahren zum Separieren von Blut.

Es gibt in der Medizin zahlreiche Anwendungsgebiete bei denen es notwendig ist, Blut des Patienten zu separieren. Beispielsweise wird bei Knochenbrüchen, bei Zahnoperationen, plastischer Chriurgie, Orthopädie und Traumatologie, sowie bei weichen Gewebsverletzungen thrombozytenreiches Blutplasma benötigt um die Heilung einzuleiten bzw. zu beschleunigen.

Es ist bereits bekannt, hierzu aus Blut des Patienten durch Zentrifugation das Blut entsprechend zu trennen.

Allerdings erfordern die Methoden zum Trennen der Anteile des Blutes erhebliches Können des jeweiligen Anwenders.

Zudem ist die Gefahr der Kontamination sehr hoch. Dokumente AU 2013 312 546 A1, EP 2 495 302 A2, US 2014 371048 A1, US2015 104824 A1 und GB 1 385 476 A gehören zum stand der Technik.

Aufgabe der Erfindung ist es daher, eine Vorrichtung und ein Verfahren vorzuschlagen, das auch von weniger versierten Anwendern genutzt werden kann und bei dem die Gefahr der Kontamination des gewonnenen Serums verringert wird.

Diese Aufgabe wird erfindungsgemäß durch eine Vorrichtung gemäß Anspruch 1 und ein Verfahren gemäß Anspruch 11 gelöst . Optionale Merkmale sind in den abhängigen Ansprüchen 2-10, 12-13 beansprucht.

Durch eine Vorrichtung gemäß Anspruch 1 kann eine sichtbare Trennung der drei Bestandteile des Blutes erzielt werden.

Dabei hat es sich als sehr vorteilhaft erwiesen, wenn das Beobachtungsrohr einen gegenüber der unteren und der oberen Kammer verjüngten Querschnitt aufweist. Damit wird das thrombozytenreiche Blutplasma gestreckt und ist leichter erkennbar. Äußerst vorteilhaft ist es gemäß einer weiteren Ausgestaltung der Erfindung auch, wenn in der unteren Kammer ein von außen bewegbarer Stempel angeordnet ist. Durch den bewegbaren Stempel können die Separationen des Blutes in der Vorrichtung derart verschoben werden, daß das thrombozytenreiche Blutplasma im Beobachtungsrohr anliegt. Dabei kann der Stempel sowohl in Richtung des Beobachtungsrohres als auch von diesem weg bewegt werden.

Sehr vorteilhaft ist es dabei, wenn der Stempel eine oder mehrere Abdichteinrichtungen gegenüber der Wandung der unteren Kammer aufweist.

Damit wird ein Austreten der beim Stempel liegenden roten Blutkörperchen verhindert. Zudem wird eine Kontamination vermieden. Eine mehrfache Abdichtung ist denkbar. Durch eine mehrfache Abdichteinrichtung wird auch sichergestellt, daß beim Zentrifugieren kein Blut austritt. Zudem wird ein Anlagern der Thrombozyten verhindert, die dann inaktiv wären.

Weiterhin ist es erfindungsgemäß sehr vorteilhaft, wenn eine Einstelleinrichtung zum Verschieben des Stempels vorgesehen ist.

Hierdurch kann der Stempel genau justiert werden.

Gemäß Erfindung, die obere Kammer ist mit einem Deckel versehen, wobei der Deckel einteilig mit der Kammer hergestellt sein kann.

Hierdurch wird die obere Kammer geschützt.

Gemäß Erfindung ist im Deckel eine Ausnehmung zum Befüllen und Entleeren der Vorrichtung vorgesehen, wobei die Ausnehmung verschließbar ausgebildet sein kann.

Hiermit wird eine einfache und sichere Möglichkeit zum Befüllen und gezielten Entleeren der Vorrichtung geschaffen. Es ist denkbar, daß die Ausnehmung mit einem Deckel oder Stopfen verschlossen werden kann.

Gemäß Erfindung ist unterhalb der Ausnehmung eine fest eingebaute als Kanüle ausgebildete Entnahme- und Befülleinrichtung vorgesehen ist, welche als Kanüle ausgebildet ist.

Damit muss nicht mit externen Kanülen oder dergleichen in die Vorrichtung eingegriffen werden. Auf diese Art und Weise wird eine Kontamination wirksam vermieden. So kann sowohl Blut in die Vorrichtung eingebracht, als auch thrombozytenreiches Plasma entnommen werden.

Gemäß Erfindung endet die Kanüle im Beobachtungsrohr.

Dadurch wird sichergestellt, daß das thrombozytenreiche Plasma gezielt entnommen werden kann.

Äußerst vorteilhaft ist es erfindungsgemäß auch, wenn eine Belüftungs- und/oder Entlüftungseinrichtung vorgesehen ist, welche mit einem Ventil ausgerüstet sein kann. Damit wird gewährleistet, daß beim Befüllen der Vorrichtung kein Überdruck und bei der Entnahme des thrombozytenreichen Plasmas kein Unterdruck in der Vorrichtung entsteht. Auch beim Verschieben der Separationen erfolgt so ein Druckausgleich. Gemäß einer weiteren Ausgestaltung der Erfindung ist es sehr vorteilhaft, wenn die Vorrichtung als Zentrifugationsgefäß ausgebildet ist.

Damit kann die Vorrichtung universell eingesetzt werden.

Sehr vorteilhaft ist es erfindungsgemäß auch, wenn die Vorrichtung aus PE, PP oder dergleichen hergestellt ist.

Durch diese Materialauswahl wird verhindert, daß sich thrombozytenreiches Plasma an den Wandungen anlagert.

Ein sehr vorteilhaftes Verfahren zum Trennen von Blut unter Nutzung der erfindungsgemäßen Vorrichtung ist im Anspruch 11 beansprucht.

Hierdurch werden klare Separationen gebildet.

Sehr vorteilhaft ist es erfindungsgemäß auch, wenn das einzubringende Blut durch eine Abwärtsbewegung des Stempels in die Vorrichtung eingesaugt wird oder daß das Blut mit einer Spritze hineingedrückt wird und ein Druckausgleich über ein Entlüftungsventil im Deckel der Vorrichtung erfolgt.

Damit kann auf einfache und sichere Weise Blut in die Vorrichtung eingebracht werden. Verunreinigungen werden vermieden.

Dabei ist es sehr vorteilhaft, wenn das thrombozytenreiche Plasma in das Beobachtungsrohr verschoben wird.

Damit kann das thrombozytenreiche Plasma sehr gut erkannt werden. Das Verschieben des thrombozytenreichen Plasmas erfolgt durch Verschieben eines Stempels.

Gemäß Erfindung wird das thrombozytenreiche Plasma über eine fest eingebaute Kanüle entnommen.

Damit kann das thrombozytenreiche Plasma sehr leicht und dennoch kontaminationsfrei entnommen werden. Durch die Kanüle kann auch das zu trennende Blut eingebracht werden, so daß ein Einreifen mit Kanülen oder dergleichen von außen vermieden wird und so Kontaminationen verhindert werden.

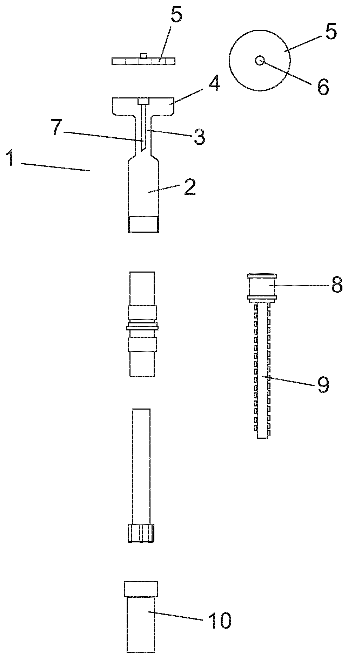

Im folgenden ist die Erfindung anhand eines Ausführungsbeispiels veranschaulicht. Hierbei zeigt die einzige Figur einen schematischen Aufbau der erfindungsgemäßen Vorrichtung zum Separieren von Blut.

Mit 1 ist eine Vorrichtung zum Separieren von Blut bezeichnet, welche als Zentrifugationsgefäß dient.

Die Vorrichtung weist eine untere Kammer 2, ein Beobachtungsrohr 3 und eine obere Kammer 4 auf, wobei das Beobachtungsrohr 3 die untere und die obere Kammer miteinander verbindet.

Die obere Kammer 4 ist mit einem aufgesetzten Deckel 5 verschlossen, in dessen Mitte eine Einfüll- und Entnahmeöffnung 6 angeordnet ist. Diese Öffnung 6 kann mit einem nicht dargestellten, geeigneten Verschluß verschlossen werden.

Unterhalb der Öffnung 6 ist eine fest eingebaute Kanüle 7 vorgesehen, welche bis in das Beobachtungsrohr 3 hinein reicht.

In der unteren Kammer 2 ist ein Stempel 8 vorgesehen, der mit einer Gewindestange 9 verbunden ist, welche über ein Griffstück 10 verstellt werden kann.

Im Deckel kann eine Belüftungs- und/oder Entlüftungseinrichtung vorgesehen sein, die mit einem Ventil ausgerüstet sein kann. Diese stellt beim Befüllen der Vorrichtung sicher, daß kein Überdruck und bei der Entnahme des thrombozytenreichen Plasmas kein Unterdruck in der Vorrichtung entsteht. Auch beim Verschieben der Separationen erfolgt hierüber ein Druckausgleich.

Zusätzlich oder alternativ kann durch eine Abwärtsbewegung des Stempels 8 Blut in die Vorrichtung gesaugt werden.

Die Zugabe von Aktivatoren oder dergleichen ist denkbar und problemlos möglich.

Durch Drehen des Griffstückes 10 kann die Position des Stempels 8 in der unteren Kammer 2 verschoben werden.

Zur Separation von Blut wird dieses durch die Öffnung 6 und die Kanüle 7 in ausreichender Menge in die Vorrichtung 1 eingefüllt.

Das eingefüllte Blut wird sodann zentrifugiert, so daß sich dieses in drei Separationen, nämlich die roten Blutkörperchen, welche sind in der unteren Kammer 2 ansammeln, thrombozytenreichs Plasma, welches sich im Beobachtungsrohr 3 ansammelt und Leukozyten, welche sich in der oberen Kammer 4 ansammeln, getrennt.

Die Menge der drei Separationen ist im wesentlichen bei jedem Blut anders, so daß für eine saubere Trennung anschließend durch Drehen des Griffstücks 10 und damit Bewegen der Position des Stempels 8 die Separationen genau den drei Abschnitten der Vorrichtung 1 zugeordnet werden.

Ist dies geschehen, kann das benötigte thrombozytenreiche Plasma über die Kanüle 7 abgezogen werden.

Wesentlich ist, daß der Stempel 8 in beide Richtungen verschoben werden kann, so daß die genaue Einstellung der Position der Separationen erfolgen kann.

Wesentlich ist, daß durch die fest eingebaute Kanüle 7 keine Notwendigkeit besteht, mit externen Kanülen oder dergleichen in die Vorrichtung 1 einzufahren, wodurch Kontamination verhindert wird. Dies gilt sowohl für das Befüllen der Vorrichtung als auch für das Entnehmen des thrombozytenreichen Plasmas.

Anwenderfehler werden weitgehend ausgeschlossen.

Die Vorrichtung wird aus PE oder PP oder anderen geeigneten Materialien hergestellt. Bei einer solchen Materialauswahl wird auch verhindert, daß sich thrombozytenreiches Plasma an den Wandungen der Vorrichtung anlagert und so inaktiv wird.

Durch diese Maßnahmen wird die Ausbeute an thrombozytenreichem Plasma erhöht.

Durch das Vermeiden von Nachfüllungen, Schütteln, Kippen und dergleichen wird die Qualität der Separation deutlich verbessert.

Die Größe der Vorrichtung 1 ist auf die zu separierende Menge Blut und deren Separationen abgestimmt.

## Patentansprüche

1. Vorrichtung zum Separieren von Blut, wobei ein Probenröhrchen mit drei Abschnitten, nämlich unterer Kammer, Beobachtungsrohr und oberer Kammer vorgesehen ist, wobei die obere Kammer mit einem Deckel versehen ist und wobei im Deckel eine Ausnehmung zum Befüllen und Entleeren der Vorrichtung vorgesehen ist, **dadurch gekennzeichnet daß** unterhalb der Ausnehmung eine fest eingebaute als Kanüle ausgebildete Entnahme- und Befülleinrichtung vorgesehen ist und daß die Kanüle im Beobachtungsrohr endet.

2. Vorrichtung nach Anspruch 1, **dadurch gekennzeichnet, daß** das Beobachtungsrohr einen gegenüber der unteren und der oberen Kammer verjüngten Querschnitt aufweist.

3. Vorrichtung nach Anspruch 1 oder 2, **dadurch gekennzeichnet, daß** in der unteren Kammer ein von außen bewegbarer Stempel angeordnet ist.

4. Vorrichtung nach Anspruch 3, **dadurch gekennzeichnet, daß** der Stempel eine oder mehrere Abdichteinrichtungen gegenüber der Wandung der unteren Kammer aufweist.

5. Vorrichtung nach Anspruch 3 oder 4, **dadurch gekennzeichnet, daß** eine Einstelleinrichtung zum Verschieben des Stempels vorgesehen ist.

6. Vorrichtung nach einem der vorangehenden Ansprüche, **dadurch gekennzeichnet, daß** der Deckel einteilig mit der Kammer hergestellt ist.

7. Vorrichtung nach Anspruch 6, **dadurch gekennzeichnet, daß** die Ausnehmung im Deckel verschließbar ausgebildet ist.

8. Vorrichtung nach einem der vorangehenden Ansprüche, **dadurch gekennzeichnet, daß** eine Belüftungs- und/oder Entlüftungseinrichtung vorgesehen ist, welche mit einem Ventil ausgerüstet sein kann.

9. Vorrichtung nach einem der vorangehenden Ansprüche, **dadurch gekennzeichnet, daß** die Vorrichtung als Zentrifugationsgefäß ausgebildet ist.

10. Vorrichtung nach einem der vorangehenden Ansprüche, **dadurch gekennzeichnet, daß** die Vorrichtung aus PE, PP oder dergleichen hergestellt ist.

11. Verfahren zum Trennen von Blut mit einer Vorrichtung nach einem der vorangehenden Ansprüche, **dadurch gekennzeichnet, daß** Blut in die Vorrichtung eingefüllt wird, dieses dann zentrifugiert wird, so daß sich rote Blutkörperchen, thrombozytenreiches Plasma und Leukozyten voneinander trennen und daß das thrombozytenreiche Plasma über die fest eingebaute Kanüle entnommen wird.

12. Verfahren nach Anspruch 11, **dadurch gekennzeichnet, daß** das einzubringende Blut durch eine Abwärtsbewegung des Stempels in die Vorrichtung eingesaugt wird oder daß das Blut mit einer Spritze hineingedrückt wird und ein Druckausgleich über ein Entlüftungsventil im Deckel der Vorrichtung erfolgt.

13. Verfahren nach Anspruch 11 oder 12, **dadurch gekennzeichnet, daß** das thrombozytenreiche Plasma in das Beobachtungsrohr verschoben wird.

## Claims

1. Device for separation of blood, wherein a sample tubelet with three sections, namely lower chamber, observation tube and upper chamber, is provided, wherein the upper chamber is provided with a cover and wherein a recess for filing and emptying the device is provided in the cover, **characterised in that** provided below the recess is a fixedly installed removing and filling device, which is configured as a cannula, and that the cannula ends in the observation tube.

2. Device according to claim 1, **characterised in that** the observation tube has a cross-section which is narrowed relative to the lower and upper chambers.

3. Device according to claim 1 or 2, **characterised in that** a plunger movable from outside is arranged in the lower chamber.

4. Device according to claim, **characterised in that** the plunger comprises one or more sealing devices relative to the wall of the lower chamber.

5. Device according to claim 3 or 4, **characterised in that** a setting device for displacing the plunger is provided.

6. Device according to any one of the preceding claims, **characterised in that** the cover is produced integrally with the chamber.

7. Device according to claim 6, **characterised in that** the recess in the cover is formed to be closable.

8. Device according to any one of the preceding claims, **characterised in that** a ventilation and/or de-aeration device, which can be equipped with a valve, is provided.

9. Device according to any one of the preceding claims, **characterised in that** the device is constructed as a centrifuge vessel.

10. Device according to any one of the preceding claims, **characterised in that** the device is produced from polyethylene, polypropylene or the like.

11. Method of separating blood by a device according to any one of the preceding claims, **characterised in that** the blood is filled into the device, this is then centrifuged so that red blood cells, platelet-rich plasma and leukocytes separate from one another and that the platelet-rich plasma is removed by way of the fixedly installed cannula.

12. Method according to claim 11, **characterised in that** the blood to be introduced is sucked in by way of a downward movement of the plunger in the device or that the blood is injected by a syringe and a pressure equalisation by way a de-aeration valve in the cover of the device is carried out.

13. Method according to claim 11 or 12, **characterised in that** the platelet-rich plasma is moved into the observation tube.

## Revendications

1. Dispositif dévolu au fractionnement du sang, dans lequel est prévu un petit tube à échantillons comprenant trois zones, à savoir une chambre inférieure, un tube d'observation et une chambre supérieure, laquelle chambre supérieure est munie d'un couvercle, un évidement affecté à l'emplissage et au vidage du dispositif étant prévu dans ledit couvercle, **caractérisé par le fait qu'**un moyen de prélèvement et d'emplissage, intégré de manière fixe et réalisé sous la forme d'une canule, est prévu au-dessous de l'évidement ; et **par le fait que** ladite canule s'achève dans le tube d'observation.

2. Dispositif selon la revendication 1, **caractérisé par le fait que** le tube d'observation présente une section transversale s'amenuisant par rapport aux chambres inférieure et supérieure.

3. Dispositif selon la revendication 1 ou 2, **caractérisé par le fait qu'**un piston, pouvant être mû de l'extérieur, est logé dans la chambre inférieure.

4. Dispositif selon la revendication 3, **caractérisé par le fait que** le piston est pourvu d'un ou de plusieurs moyen(s) assurant l'étanchéité par rapport à la paroi de la chambre inférieure.

5. Dispositif selon la revendication 3 ou 4, **caractérisé par le fait qu'**un moyen de réglage est prévu pour déplacer le piston.

6. Dispositif selon l'une des revendications précédentes, **caractérisé par le fait que** le couvercle est produit d'un seul tenant avec la chambre.

7. Dispositif selon l'une des revendications précédentes, **caractérisé par le fait que** l'évidement pratiqué dans le couvercle est conçu avec faculté d'obturation.

8. Dispositif selon l'une des revendications précédentes, **caractérisé par le fait qu'**il est prévu un moyen d'aération et/de ventilation pouvant être équipé d'une soupape.

9. Dispositif selon l'une des revendications précédentes, **caractérisé par le fait que** ledit dispositif est réalisé sous la forme d'un récipient de centrifugation.

10. Dispositif selon l'une des revendications précédentes, **caractérisé par le fait que** ledit dispositif est fabriqué en du PE, en du PP ou en une substance similaire.

11. Procédé de fractionnement du sang à l'aide d'un dispositif conforme à l'une des revendications précédentes, **caractérisé par le fait que** du sang est déversé dans le dispositif, pour être ensuite centrifugé de façon telle que des globules rouges, du plasma riche en plaquettes et des leucocytes se dissocient les uns des autres ; et **par le fait que** ledit plasma riche en plaquettes est prélevé par l'intermédiaire de la canule à intégration fixe.

12. Procédé selon la revendication 11, **caractérisé par le fait que** le sang à introduire est aspiré par un mouvement descendant faisant pénétrer le piston dans le dispositif ; ou **par le fait que** le sang est injecté sous pression à l'aide d'une seringue, et un équilibrage de pression est opéré par l'intermédiaire d'une soupape de ventilation implantée dans le couvercle dudit dispositif.

13. Procédé selon la revendication 11 ou 12, **caractérisé par le fait que** le plasma riche en plaquettes est transféré dans le tube d'observation.
